# EUROPEAN PATENT APPLICATION

(11) **EP 3 510 857 A1**
(43) Date of publication of application: **17.07.2019**
(21) Application number: 17848796.3
(22) Date of filing: 06.09.2017
(51) Int. Cl.: A01G 7/06, A01G 2/00, C12N 1/21, C12N 7/01

(54) **GLOWING PLANT**

(30) Priority: 06.09.2016 JP 2016173930
(71) Applicant: NEC Solution Innovators, Ltd., Tokyo 136-8627 (JP)
(72) Inventor: WAGA, Iwao, Tokyo 136-8627 (JP); ISHIWATA, Ayako, Tokyo 136-8627 (JP); SHIRATORI, Ikuo, Tokyo 136-8627 (JP); SHIMIZU, Akihisa, Tokyo 136-8627 (JP); MISHIMA, Hiroshi, Tokyo 136-8627 (JP)
(74) Representative: Vossius & Partner Patentanwälte Rechtsanwälte mbB
(86) International application number: PCT/JP2017/032095
(87) International publication number: WO 2018/047858

(57) **Abstract**

The present invention provides a new glowing plant. The glowing plant of the present invention includes a fluorescent protein applied to a surface of the plant; or a fluorescent protein absorbed inside the plant. The plant is preferably a flower.

SEQUENCE LISTING

<110>  NEC Solution Innovators, Ltd.

<120>  luminescent plant

<130>  1010051266

<150>  JP 2016-173930
<151>  2016-09-06

<160>  2

<170>  PatentIn version 3.5

<210>  1
<211>  219
<212>  PRT
<213>  Artificial Sequence

<220>
<223>  fluorescence protein

<400>  1

Met Thr Thr Phe Lys Ile Glu Ser Arg Ile His Gly Asn Leu Asn Gly
1               5                   10                  15

Glu Lys Phe Glu Leu Val Gly Gly Gly Val Gly Glu Glu Gly Arg Leu
            20                  25                  30

Glu Ile Glu Met Lys Thr Lys Asp Lys Pro Leu Ala Phe Ser Pro Phe
        35                  40                  45

Leu Leu Ser His Cys Met Gly Tyr Gly Phe Tyr His Phe Ala Ser Phe
    50                  55                  60

Pro Lys Gly Thr Lys Asn Ile Tyr Leu His Ala Ala Thr Asn Gly Gly
65                  70                  75                  80

Tyr Thr Asn Thr Arg Lys Glu Ile Tyr Glu Asp Gly Gly Ile Leu Glu
                85                  90                  95

Val Asn Phe Arg Tyr Thr Tyr Glu Phe Asn Lys Ile Ile Gly Asp Val
            100                 105                 110

Glu Cys Ile Gly His Gly Phe Pro Ser Gln Ser Pro Ile Phe Lys Asp
        115                 120                 125

Thr Ile Val Lys Thr Cys Pro Thr Val Asp Leu Met Leu Pro Met Ser
    130                 135                 140

Gly Asn Ile Ile Ala Ser Ser Tyr Ala Lys Ala Phe Gln Leu Lys Asp
145                 150                 155                 160

Gly Ser Phe Tyr Thr Ala Glu Val Lys Asn Asn Ile Asp Phe Lys Asn
                165                 170                 175

Pro Ile His Glu Ser Phe Ser Lys Ser Gly Pro Met Phe Thr His Arg
            180                 185                 190

Arg Val Glu Glu Thr His Thr Lys Glu Asn Leu Ala Met Val Glu Tyr
        195                 200                 205

Gln Gln Val Phe Asn Ser Ala Pro Arg Asp Met
    210                 215

<210>  2
<211>  219
<212>  PRT
<213>  Artificial Sequence

<220>
<223>  fluorescence protein

<400>  2

Met Thr Thr Phe Lys Ile Glu Ser Arg Ile His Gly Asn Leu Asn Gly
1               5                   10                  15

Glu Lys Phe Glu Leu Val Gly Gly Gly Val Gly Glu Glu Gly Arg Leu
            20                  25                  30

Glu Ile Glu Met Lys Thr Lys Asp Lys Pro Leu Ala Phe Ser Pro Phe
        35                  40                  45

Leu Leu Thr Thr Cys Met Gly Tyr Gly Phe Tyr His Phe Ala Ser Phe
    50                  55                  60

Pro Lys Gly Ile Lys Asn Ile Tyr Leu His Ala Ala Thr Asn Gly Gly
65                  70                  75                  80

Tyr Thr Asn Thr Arg Lys Glu Ile Tyr Glu Asp Gly Gly Ile Leu Glu
                85                  90                  95

Val Asn Phe Arg Tyr Thr Tyr Glu Phe Asn Lys Ile Ile Gly Asp Val
            100                 105                 110

Glu Cys Ile Gly His Gly Phe Pro Ser Gln Ser Pro Ile Phe Lys Asp
        115                 120                 125

Thr Ile Val Lys Ser Cys Pro Thr Val Asp Leu Met Leu Pro Met Ser
    130                 135                 140

Gly Asn Ile Ile Ala Ser Ser Tyr Ala Tyr Ala Phe Gln Leu Lys Asp
145                 150                 155                 160

Gly Ser Phe Tyr Thr Ala Glu Val Lys Asn Asn Ile Asp Phe Lys Asn
                165                 170                 175

Pro Ile His Glu Ser Phe Ser Lys Ser Gly Pro Met Phe Thr His Arg
            180                 185                 190

Arg Val Glu Glu Thr Leu Thr Lys Glu Asn Leu Ala Ile Val Glu Tyr
        195                 200                 205

Gln Gln Val Phe Asn Ser Ala Pro Arg Asp Met
    210                 215

SEQUENCE LISTING

<110>  NEC Solution Innovators, Ltd.

<120>  Glowing plant

<130>  AC1238 EP S3

<140>  EP 17 84 8796.3
<141>  2017-09-06

<150>  JP 2016-173930
<151>  2016-09-06

<160>  2

<170>  PatentIn version 3.5

<210>  1
<211>  219
<212>  PRT
<213>  Artificial Sequence

<220>
<223>  fluorescence protein

<400>  1

Met Thr Thr Phe Lys Ile Glu Ser Arg Ile His Gly Asn Leu Asn Gly
1               5                   10                  15

Glu Lys Phe Glu Leu Val Gly Gly Gly Val Gly Glu Glu Gly Arg Leu
            20                  25                  30

Glu Ile Glu Met Lys Thr Lys Asp Lys Pro Leu Ala Phe Ser Pro Phe
        35                  40                  45

Leu Leu Ser His Cys Met Gly Tyr Gly Phe Tyr His Phe Ala Ser Phe
    50                  55                  60

Pro Lys Gly Thr Lys Asn Ile Tyr Leu His Ala Ala Thr Asn Gly Gly
65                  70                  75                  80

Tyr Thr Asn Thr Arg Lys Glu Ile Tyr Glu Asp Gly Gly Ile Leu Glu
                85                  90                  95

Val Asn Phe Arg Tyr Thr Tyr Glu Phe Asn Lys Ile Ile Gly Asp Val
            100                 105                 110

Glu Cys Ile Gly His Gly Phe Pro Ser Gln Ser Pro Ile Phe Lys Asp
        115                 120                 125

Thr Ile Val Lys Thr Cys Pro Thr Val Asp Leu Met Leu Pro Met Ser
    130                 135                 140

Gly Asn Ile Ile Ala Ser Ser Tyr Ala Lys Ala Phe Gln Leu Lys Asp
145                 150                 155                 160

Gly Ser Phe Tyr Thr Ala Glu Val Lys Asn Asn Ile Asp Phe Lys Asn
                165                 170                 175

Pro Ile His Glu Ser Phe Ser Lys Ser Gly Pro Met Phe Thr His Arg
            180                 185                 190

Arg Val Glu Glu Thr His Thr Lys Glu Asn Leu Ala Met Val Glu Tyr
        195                 200                 205

Gln Gln Val Phe Asn Ser Ala Pro Arg Asp Met
    210                 215

<210>  2
<211>  219
<212>  PRT
<213>  Artificial Sequence

<220>
<223>  fluorescence protein

<400>  2

Met Thr Thr Phe Lys Ile Glu Ser Arg Ile His Gly Asn Leu Asn Gly
1               5                   10                  15

Glu Lys Phe Glu Leu Val Gly Gly Gly Val Gly Glu Glu Gly Arg Leu
            20                  25                  30

Glu Ile Glu Met Lys Thr Lys Asp Lys Pro Leu Ala Phe Ser Pro Phe
        35                  40                  45

Leu Leu Thr Thr Cys Met Gly Tyr Gly Phe Tyr His Phe Ala Ser Phe
    50                  55                  60

Pro Lys Gly Ile Lys Asn Ile Tyr Leu His Ala Ala Thr Asn Gly Gly
65                  70                  75                  80

Tyr Thr Asn Thr Arg Lys Glu Ile Tyr Glu Asp Gly Gly Ile Leu Glu
                85                  90                  95

Val Asn Phe Arg Tyr Thr Tyr Glu Phe Asn Lys Ile Ile Gly Asp Val
            100                 105                 110

Glu Cys Ile Gly His Gly Phe Pro Ser Gln Ser Pro Ile Phe Lys Asp
        115                 120                 125

Thr Ile Val Lys Ser Cys Pro Thr Val Asp Leu Met Leu Pro Met Ser
    130                 135                 140

Gly Asn Ile Ile Ala Ser Ser Tyr Ala Tyr Ala Phe Gln Leu Lys Asp
145                 150                 155                 160

Gly Ser Phe Tyr Thr Ala Glu Val Lys Asn Asn Ile Asp Phe Lys Asn
                165                 170                 175

Pro Ile His Glu Ser Phe Ser Lys Ser Gly Pro Met Phe Thr His Arg
            180                 185                 190

Arg Val Glu Glu Thr Leu Thr Lys Glu Asn Leu Ala Ile Val Glu Tyr
        195                 200                 205

Gln Gln Val Phe Asn Ser Ala Pro Arg Asp Met
    210                 215

## Description

### TECHNICAL FIELD

The present invention relates to a glowing plant.

### BACKGROUND ART

Heretofore, a technique for making a glowing flower by introducing a fluorescent protein into a flower has been developed and achieved (Patent Literature 1). However, since a gene recombination technique is used for the glowing flower, there are regulations on cultivation and the like in some countries due to concerns about the influence on the natural world.

### Citation List

### Patent Literature

Patent Literature 1: JP2008-22817A

### SUMMARY OF INVENTION

### Technical Problem

Therefore, as a method of causing the flower to emit light, for example, a method of applying an organic synthetic fluorescent compound that emits fluorescence to a plant to impart fluorescence to the flower is considered. However, since the compound to be applied to the flower is an organic compound, there is a concern about the influence on the natural world. In addition, when the organic synthetic compound is applied to a growing flower, the growth of the flower may be hindered.

Accordingly, the present invention is intended to provide a new glowing plant having high environmental and plant safety.

### Solution to Problem

In order to achieve the above object, the present invention provides a glowing plant including: a fluorescent protein applied to a surface of the plant; or a fluorescent protein absorbed inside the plant.

The present invention also provides a method of producing a glowing plant, including the step of: applying a fluorescent protein to a surface of the plant; or causing a fluorescent protein to be absorbed inside the plant.

### Advantageous Effects of Invention

According to the present invention, for example, a plant that emits fluorescence can be obtained without using a gene recombination technique for the plant. In addition, since a fluorescent protein is applied to a plant, for example, as compared to the case of applying an organic synthetic fluorescent compound described above, the affinity to plants is high, and in the case of a biodegradable fluorescent protein, concerns about persistence are removed, and therefore, the safety to plants and environments is high.

### BRIEF DESCRIPTION OF DRAWINGS

FIG. 1 shows photographs of the roses after application of a fluorescent protein in Example 1-1 of the present invention.
FIG. 2 shows photographs of the Phalaenopsis orchids after application of a fluorescent protein in Example 1-1 of the present invention.
FIG. 3 shows photographs of the roses after application of a fluorescent protein in Example 1-2 of the present invention.
FIG. 4 shows photographs of the roses after application of a fluorescent protein in Example 1-3 of the present invention.
FIG. 5 shows photographs of the Phalaenopsis orchids after application of a fluorescent protein in Example 1-3 of the present invention.
FIG. 6 shows photographs of the roses after absorption of a fluorescent protein in Example 2-1 of the present invention.

### DESCRIPTION OF EMBODIMENTS

In the glowing plant of the present invention, for example, the plant is a flower.

The glowing plant of the present invention includes, for example, a fluorescent protein absorbed from a root or a stem.

In the glowing plant of the present invention, for example, the fluorescent protein applied to the surface of the plant is a fluorescent protein-containing substance or the fluorescent protein absorbed by the plant is a fluorescent protein-containing substance, and the fluorescent protein-containing substance is a homogenate of a host expressing the fluorescent protein.

In the glowing plant of the present invention, for example, the fluorescent protein-containing substance is an individual-removed substance obtained by removing an individual of the host from the homogenate.

In the glowing plant of the present invention, for example, the fluorescent protein applied to the surface of the plant is a fluorescent protein-containing substance or the fluorescent protein absorbed by the plant is a fluorescent protein-containing substance, and the fluorescent protein-containing substance is a culture of a host expressing the fluorescent protein.

In the glowing plant of the present invention, for example, the fluorescent protein-containing substance is a supernatant of the culture.

In the glowing plant of the present invention, for example, the host is a microorganism or a virus.

In the method of producing a glowing plant of the present invention, for example, the plant is a flower.

In the method of producing a glowing plant of the present invention, for example, in the absorption step, a root or a stem of the plant is brought into contact with water that contains the fluorescent protein to cause the fluorescent protein to be absorbed from the root or stem.

In the method of producing a glowing plant of the present invention, for example, in the application step, a fluorescent protein-containing substance that contains the fluorescent protein is applied to the surface of the plant, or in the absorption step, the fluorescent protein-containing substance that contains the fluorescent protein is caused to be absorbed by the plant, and the fluorescent protein-containing substance is a homogenate of a host expressing the fluorescent protein.

In the method of producing a glowing plant of the present invention, for example, the fluorescent protein-containing substance is an individual-removed substance obtained by removing an individual of the host from the homogenate.

The method of producing a glowing plant of the present invention further includes, for example, the step of filtrating the homogenate with a filter through which the host does not pass to obtain the individual-removed substance.

In the method of producing a glowing plant of the present invention, for example, in the application step, the fluorescent protein-containing substance that contains the fluorescent protein is applied to the surface of the plant, or in the absorption step, the fluorescent protein-containing substance that contains the fluorescent protein is caused to be absorbed by the plant, and the fluorescent protein-containing substance is a culture of a host expressing the fluorescent protein.

In the method of producing a glowing plant of the present invention, for example, the fluorescent protein-containing substance is a supernatant of the culture.

The method of producing a glowing plant of the present invention further includes, for example, the step of purifying the fluorescent protein from the fluorescent protein-containing substance, wherein in the application step, the purified fluorescent protein is applied.

In the method of producing a glowing plant of the present invention, for example, in the purification step, the fluorescent protein is purified using a column.

In the method of producing a glowing plant of the present invention, for example, the host is a microorganism or a virus.

The glowing plant of the present invention is characterized in that, as described above, it includes a fluorescent protein applied to a surface of the plant or a fluorescent protein absorbed inside the plant. In the present invention, other configurations and conditions are not particularly limited. Regarding the description of the glowing plant of the present invention, reference can be made to the description as to the method of producing a glowing plant of the present invention to be described below.

In the glowing plant of the present invention, the plant is not particularly limited, and examples thereof include rose, carnation, chrysanthemum, Annual Baby's-breath, gerbera, lily, and orchid.

In the glowing plant of the present invention, for example, the fluorescent protein may be applied to the whole plant or to a part of the plant. Furthermore, in the glowing plant of the present invention, for example, the fluorescent protein may be absorbed by the whole plant or by a part of the plant. In the cases where the fluorescent protein is applied to a part of the plant or the fluorescent protein is absorbed by a part of the plant, examples of the part of the plant include a flower, a leaf, a branch, a fruit, a sepal, a pistil, and a stamen. The application or absorption of the fluorescent protein may be performed during cultivation or after harvesting, for example.

In the glowing plant of the present invention, the plant does not have, for example, genes encoding the fluorescent protein.

In the glowing plant of the present invention, the absorbed fluorescent protein is present, for example, in a vessel of the plant.

In the present invention, the fluorescent protein is not particularly limited, and examples thereof include GFP (Green Fluorescent Protein), YFP (Yellow Fluorescent Protein), RFP (Red Fluorescent Protein), and fluorescent proteins described in Japanese Unexamined Patent Application No. 2008-22817, Japanese Patent Application No. 2016-092095, and Reference Literature 1 described below. In addition, examples of the fluorescent protein include a blue light type fluorescent protein and a black light type fluorescent protein. Examples of the blue light type fluorescent protein include eYGFP (enhanced yellow-green fluorescent protein, accession NO. #LC21752) and EGFP (enhanced green fluorescent protein). Examples of the black light type fluorescent protein include eYGFPuv (enhanced yellow-green fluorescent protein uv, accession NO. #LC217533), and GFPuv (Green fluorescent protein uv). For example, only one type of the fluorescent proteins may be used alone, or two or more types of them may be used in combination.

### Reference Literature 1: Shimizu A et al. PLoS One. 2017 Jul 11;12(7)

The fluorescent protein can be obtained, for example, from a host expressing the fluorescent protein. The host is, for example, a microorganism or a virus. The microorganism is, for example, E.coli.

In the present invention, the fluorescent protein is, for example, a purified fluorescent protein. The fluorescent protein may be used alone or a composition that contains the fluorescent protein (fluorescent protein-containing substance) may be used.

The fluorescent protein-containing substance may be, for example, a culture of a host expressing the fluorescent protein. When the fluorescent protein expressed in the host is an extracellular protein, the fluorescent protein-containing substance may be, for example, the culture as it or a supernatant obtained by removing the host individual from the culture. On the other hand, when the fluorescent protein expressed in the host is an intracellular protein, the fluorescent protein-containing substance is preferably, for example, a homogenate of the host. The fluorescent protein may be, for example, the homogenate as it is, or may be an individual-removed substance obtained by removing the remaining host individual from the homogenate. For example, regardless of whether the fluorescent protein-containing substance is the intracellular protein or the extracellular protein, the host individual is preferably removed. The host individual can be removed by, for example, filtrating the culture or the homogenate with a filter through which the host individual does not pass. By removing the host individual in this manner, for example, it is possible to prevent the host expressing a fluorescent protein using a genetic recombination technique from being contained in the fluorescent protein-containing substance. Thus, for example, even when the fluorescent protein is prepared from the recombinant host, the host, which is a genetically modified product, can be prevented from diffusing or contaminating during the distribution of the glowing plant.

The purified fluorescent protein can be obtained, for example, by applying a purification treatment to the culture, the supernatant of the culture, the homogenate, and the like. The purification treatment is not particularly limited, and can be performed by, for example, known methods for extracting proteins, and specific examples thereof include the above describe removal of the host individual, salting-out, and column-purification. The combinations, conditions, and the like of the purification treatment are not particularly limited, and can be appropriately determined according to the type of the fluorescent protein, the type of the host, and the like.

The fluorescent protein may be in the state of a liquid or may be in the state of a solid such as a powder, for example. In the former case, examples of the fluorescent protein include the culture, the supernatant, and the homogenate. In the latter case, examples of the fluorescent protein include purified fluorescent protein and partially purified fluorescent protein. In the case of applying the fluorescent protein to a plant or causing the fluorescent protein to be absorbed by the plant, the fluorescent protein may be used as a mixed liquid of the fluorescent protein (application liquid or absorption liquid) obtained by mixing the fluorescent protein with a solvent or the like. The fluorescent protein may be dissolved or dispersed in the solvent, for example. The solvent is not particularly limited, and examples thereof include a phosphoric acid buffer, a Tris buffer, and a HEPES buffer.

The fluorescent protein-containing substance may further include additives, for example, and examples of the additive include surfactants and starch pastes. The surfactant may be, for example, a nonionic surfactant such as Tween 20. Owing to the surfactant, for example, the fluorescent protein-containing substance on the surface of the plant can be prevented from being repelled when applied to the plant so that the adhesion of the fluorescent protein-containing substance can be improved. It is presumed that this is because the fluorescent protein-containing substance and the oil component on the surface of the plant become intimate owing to the surfactant as well as the surfactant destructs the cell on the surface of the plant so that the fluorescent protein-containing substance is easily absorbed by the plant. The present invention, however, is not limited to this presumption. Furthermore, owing to the starch paste, for example, the fluorescent protein-containing substance can sufficiently prevent the fluorescent protein from drying out and can sufficiently prevent the fluorescent discoloration due to the drying when applied to the plant.

As described above, the method of producing a glowing plant of the present invention is characterized in that it includes the step of applying a fluorescent protein to the surface of the plant or causing a fluorescent protein to be absorbed inside the plant. In the present invention, other configurations and conditions are not particularly limited. Regarding the description of the method of producing a glowing plant of the present invention, for example, reference can be made to the description as to the glowing plant of the present invention.

In the application step, the amount of the fluorescent protein to be applied to the plant is not particularly limited, and may be appropriately determined according to, for example, the type of the plant, the target area of the plant to be applied with the fluorescent protein, the type of the fluorescent protein, and the like. The amount of the fluorescent protein to be applied is, for example, 0.01 mg to 1 mg per square centimeter.

The method of applying the fluorescent protein in the application step is not particularly limited, and examples thereof include spraying of the fluorescent protein with a spray or the like, application of the fluorescent protein with a brush or the like, sprinkling of powders that contain the fluorescent protein, and immersion in a liquid that contains the fluorescent protein.

The content of the fluorescent protein in the application liquid is not particularly limited. When the application liquid contains the additive, the ratio of the additive to the fluorescent protein is not particularly limited. As a specific example, the surfactant can be added to a protein solution of 1 mg/ml in a phosphate buffer so as to be 1 % w/v, for example. The starch paste may be added to a protein solution of 1 mg/ml in a phosphoric acid buffer so as to be 50% v/v, for example. The conditions of the application liquid are not particularly limited, and the pH is, for example, 7.4 to 8.0.

The method of causing the fluorescent protein to be absorbed in the absorption step is not particularly limited, and for example, the absorption liquid can be absorbed from the root or stem of the plant. The duration for causing the fluorescent protein to be absorbed is not particularly limited, and is, for example, 1 to 2 days or 2 days.

The content of the fluorescent protein in the absorption liquid is not particularly limited. The conditions of the absorption liquid are not particularly limited, and the pH is, for example, 7.4 to 8.0.

When the fluorescent protein in a liquid state is applied to a plant or is caused to be absorbed by a plant in the application step or the absorption step, for example, an application liquid or an absorption liquid that contains the fluorescent protein is used. The application liquid or the absorption liquid can be any liquid as long as it contains the fluorescent protein and is in a liquid state. The application liquid or the absorption liquid may be, for example, the fluorescent protein-containing substance described above. When the fluorescent protein-containing substance is in a liquid state, for example, this may be used as the application liquid or the absorption liquid, or the application liquid or the absorption liquid may be prepared by mixing the fluorescent protein or the protein-containing substance and optionally the additive with the solvent.

The type of the fluorescent protein contained in the application liquid or the absorption liquid is not particularly limited, and, for example, the application liquid or the absorption liquid may contain one type or two or more types of the fluorescent protein.

### Examples

Examples of the present invention are described below. The present invention, however, is not limited by the following examples. Commercially available reagents were used based on these protocols unless otherwise stated.

### Example 1

It was examined that a glowing plant was obtained by applying a fluorescent protein to the surface of a plant.

### (Example 1-1)

### (1) Fluorescent protein liquid

A recombinant protein (blue light type, SEQ ID NO: 1) to which a His-tag has been added at the C-end was expressed in E.coli (DH5a) by a conventional method, and a supernatant that contains the recombinant protein was recovered from the homogenate of the E.coli. The supernatant was subjected to an affinity column (Ni column) to bind the recombinant protein in the supernatant to the column, the recombinant protein was eluted with 0.2 mol/L imidazole, and a fraction containing the recombinant protein was recovered. Next, the recovered fraction was subjected to a gel filtration column (PD10 column, GE healthcare), eluted with a phosphoric acid buffer containing no NaCl, and the fraction containing the recombinant protein was recovered as a purified fraction. This purified fraction was used as a protein liquid in the following step.
SEQ ID NO:1:

MTTFKIESRIHGNLNGEKFELVGGGVGEEGRLEIEMKTKDKPLAFSPFLLSHCMGYGFYH
FASFPKGTKNIYLHAATNGGYTNTRKEIYEDGGILEVNFRYTYEFNKIIGDVECIGHGFPS
QSPIFKDTIVKTCPTVDLMLPMSGNIIASSYAKAFQLKDGSFYTAEVKNNIDFKNPIHESFS
KSGPMFTHRRVEETHTKENLAMVEYQQVFNSAPRDM

### (2) Plant

Commercially available white rose and Phalaenopsis orchid V3 were used.

### (3) Application

Tween 20 was added to the protein liquid (1.3 mg/ml) so as to achieve the final concentration of 5%, thereby preparing an application liquid. The application liquid was applied to the petals of the plants by a brush. The application amount to the petal was about 0.2 mL per flower.

### (4) Photographing

The plant to which the application liquid has been applied was put in a box the inside of which was painted black, so that natural light from the outside did not enter. Then, the plant was photographed under the bright field condition and under the excitation light irradiation condition, and the color and fluorescence of the petal of the plant was examined. The light irradiation condition was as follows. In the bright field condition, the plant was irradiated with light under a white fluorescent lamp. In the excitation light irradiation condition, the plant was irradiated with excitation light having a maximum value at 470 nm using a floodlight (VBL-S150, Valolr Corporation) equipped with a long wavelength cut filter (SV0490, Asahi Spectra Co., Ltd.). A digital camera (Eos Kiss x7i, Canon Inc.) was used as the camera. In the bright field condition, the photographing was performed in both a state in which a viewing filter (SC-52, FUJIFILM Corporation) for cutting blue light from the light source was attached to the lens of the camera and a state in which the viewing filter was not attached to the lens of the camera.

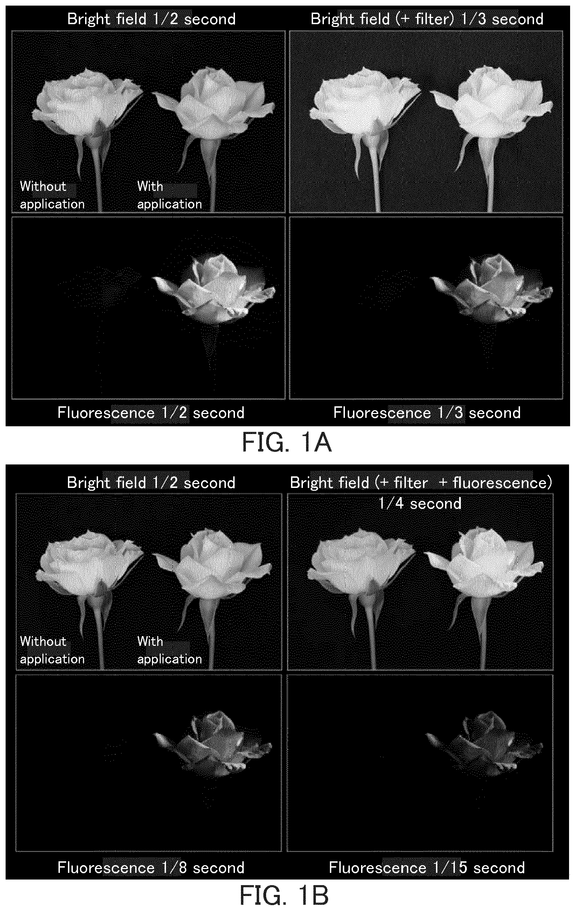

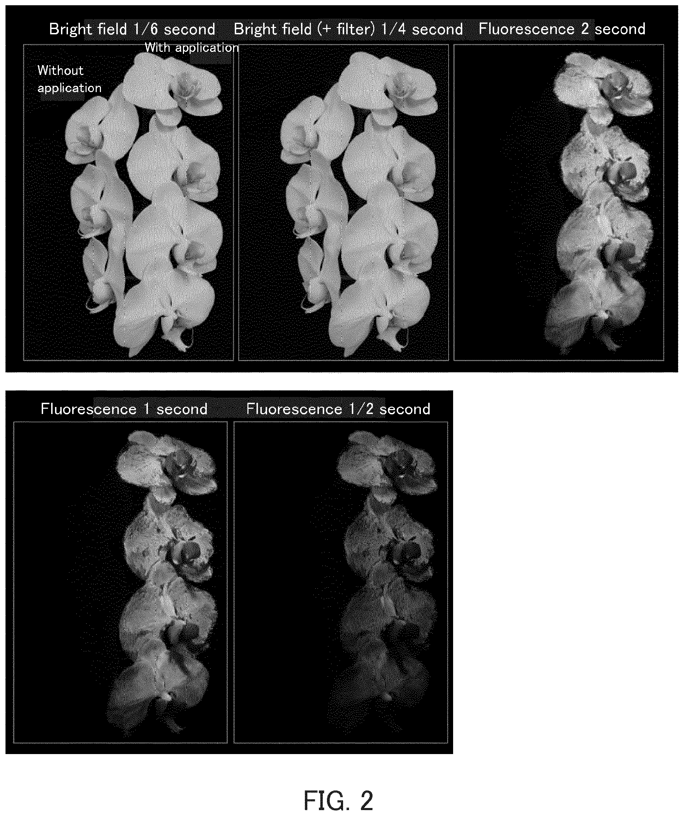

The results are shown in FIGs. 1 and 2. FIG. 1 shows photographs of the roses after the application of the application liquid. In (A) of FIG. 1, the upper left is a photograph taken under the conditions of the bright field (without viewing filter) and the exposure time of 1/2 second, the upper right is a photograph taken under the conditions of the bright field (with viewing filter) and the exposure time of 1/3 second, the lower left is a photograph taken under the conditions of the excitation light irradiation and the exposure time of 1/2 second, and the lower right is a photograph taken under the conditions of the excitation light irradiation and the exposure time of 1/3 second. In (B) of FIG. 1, the upper left is a photograph taken under the conditions of the bright field (without viewing filter) and the exposure time of 1/2 second, the upper right is a photograph taken under the conditions of the bright field and the excitation light irradiation (with viewing filter) and the exposure time of 1/4 second, the lower left is a photograph taken under the conditions of the excitation light irradiation and the exposure time of 1/8 second, and the lower right is a photograph taken under the conditions of the excitation light irradiation and the exposure time of 1/15 second. In each photograph, the rose on the right side is a rose (with application) the petals of which have been applied with the application liquid, and the rose on the left side is a rose (without application) to which the application liquid has not been applied.

In (A) of FIG. 1, in the upper left photograph which is taken under the conditions of the bright field without the viewing filter, the petals exhibited a non-fluorescent white color both with and without application of the application liquid. In the upper right photograph which is taken under the conditions of the bright field with the viewing filter, the petals exhibited a non-fluorescent yellow color due to the cutting of blue light from the light source by the viewing filter, both with and without application of the application liquid. In the lower left and lower right photographs which are taken under the excitation light irradiation condition, the rose without application could not be visually recognized in the photograph in the box, whereas the petals of the rose with application exhibited a green fluorescent color. From these results, it was confirmed that the plant emits fluorescence by the application of the fluorescent protein.

In (B) of FIG. 1, the upper left photograph which is taken under the conditions of the bright field without the viewing filter, and the lower left and lower right photographs which are taken under the excitation light irradiation condition showed the same results as in (A) of FIG. 1. In the upper right photograph taken under the conditions of the bright field with the viewing filter and the excitation light irradiation, the petals of the rose without application showed a non-fluorescent yellow color as in the upper right photograph of (A) of FIG. 1, whereas the petals of the rose with application exhibited a fluorescent yellow color. From these results, it was confirmed that the plant emits fluorescence even in the bright field by the application of the fluorescent protein.

FIG. 2 shows photographs of the Phalaenopsis orchids after the application of the application liquid. In FIG. 2, the upper left is a photograph taken under the conditions of the bright field (without viewing filter) and the exposure time of 1/6 second. The upper middle is a photograph taken under the conditions of the bright field (with viewing filter) and the exposure time of 1/4 second. The upper right is a photograph taken under the conditions of the excitation light irradiation and the exposure time of 2 seconds. The lower left is a photograph taken under the conditions of the excitation light irradiation and the exposure time of 1 second. The lower middle is a photograph taken under the conditions of the excitation light irradiation and the exposure time of 1/2 second. In each photograph, the flowers in the right row are flowers (with application) to which the application liquid has been applied, and the flowers in the left row are flowers (without application) to which the application liquid has not been applied.

In FIG. 2, in the upper left photograph which is taken under the conditions of the bright field without the filter, the petals exhibited a non-fluorescent white color both with and without application of the application liquid. In the upper middle photograph which is taken under the conditions of the bright field with the filter, the petals exhibited a non-fluorescent yellow color due to the cutting of blue light from the light source by the viewing filter, both with and without application of the application liquid. In the upper right, lower left, and lower middle photographs which are taken under the excitation light irradiation condition, the flowers without application could not be visually recognized in the photographs in the box, whereas the petals of the flowers with application exhibited a green fluorescent color. From these results, it was confirmed that the plant emits fluorescence by the application of the fluorescent protein.

### (Example 1-2)

A recombinant protein (black light type, SEQ ID NO: 2) to which a His-tag has been added at the C-end was expressed in E.coli (DH5a) in the same manner as in Examples 1-1. Then, a protein liquid was obtained in the same manner as in Example 1-1. Tween 20 was added to the protein liquid (0.12 mg/ml) so as to achieve the final concentration of 5%, thereby preparing an application liquid. The application liquid was applied to the plant in the same manner as in Example 1-1.
SEQ ID NO:2:

MTTFKIESRIHGNLNGEKFELVGGGVGEEGRLEIEMKTKDKPLAFSPFLLTTCMGYGFYH
FASFPKGIKNIYLHAATNGGYTNTRKEIYEDGGILEVNFRYTYEFNKIIGDVECIGHGFPSQ
SPIFKDTIVKSCPTVDLMLPMSGNIIASSYAYAFQLKDGSFYTAEVKNNIDFKNPIHESFSK
SGPMFTHRRVEETLTKENLAIVEYQQVFNSAPRDM

A UV LED floodlight (NS365-FLB-30WR, NITRIDE) equipped with a visible-light absorption filter (UL-360, OMG) was used as a light source for the light irradiation. For photographing, the same camera as in Example 1-1 was used without attaching a viewing filter to the lens. Except for these points, the plant was photographed in the same manner as in Example 1-1.

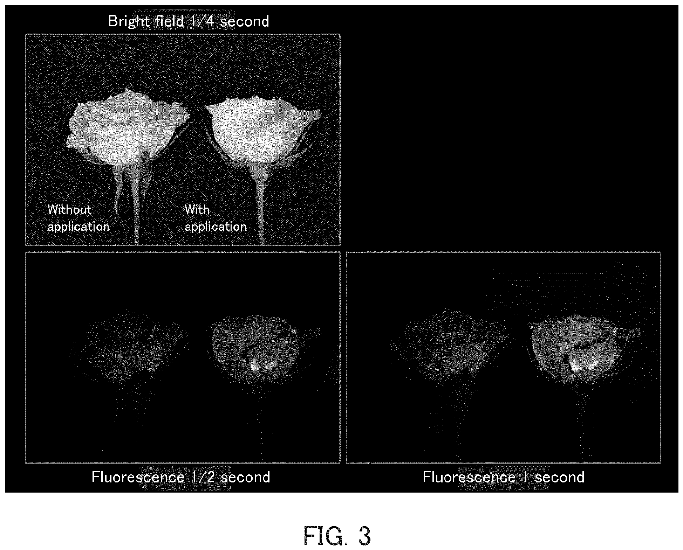

The results are shown in FIG. 3. FIG. 3 shows photographs of the roses after the application of the application liquid. In FIG. 3, the upper left is a photograph taken under the conditions of the bright field and the exposure time of 1/4 second, the lower left is a photograph taken under the condition of the exposure time of 1/2 second, and the lower right is a photograph taken under the condition of the exposure time of 1/3 second. In each photograph, the rose on the right side is a rose (with application) the petals of which have been applied with the application liquid, and the rose on the left side is a rose (without application) to which the application liquid has not been applied.

In FIG. 3, in the upper left photograph which is taken under the bright field condition, the petals exhibited a non-fluorescent white color both with and without application of the application liquid. In the lower left and lower right photographs of FIG. 3 which are taken under the excitation light irradiation condition, the rose without application could be visually recognized with a part of the petals exhibiting a slight blue color, however this is considered to be a background including extremely weak intrinsic fluorescence of the plant and not fluorescence. In contrast, the petals of the rose with application exhibited a green fluorescent color. From these results, it was confirmed that the plant emits fluorescence by the application of the fluorescent protein.

### (Examples 1-3)

Tween 20 was added to the protein liquid (0.12 mg/ml) prepared in Examples 1-2 so as to achieve the final concentration of 5%, and an equal volume of starch paste (commercially available) was added to the protein liquid, thereby preparing an application liquid. Except for this point, the fluorescent protein liquid was applied to the plant and photographing was performed in the same manner as in Examples 1-2.

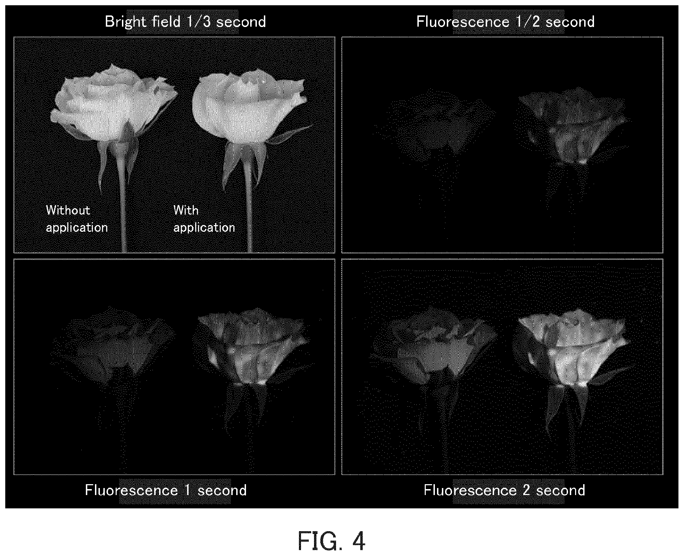

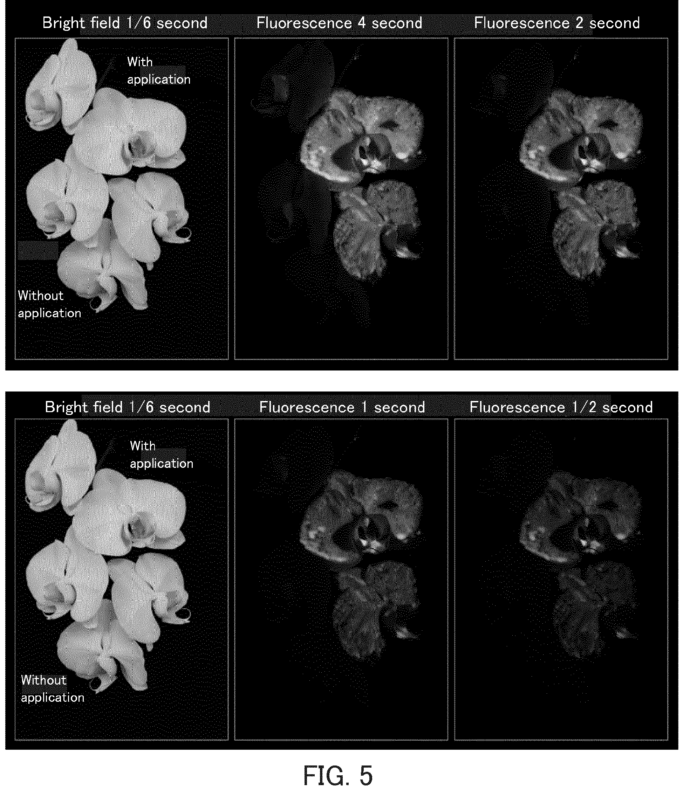

The results are shown in FIGs. 4 and 5. FIG. 4 shows photographs of the roses after the application of the application liquid. In FIG. 4, the upper left is a photograph taken under the conditions of the bright field and the exposure time of 1/3 second, the upper right is a photograph taken under the condition of the exposure time of 1/2 second, the lower left is a photograph taken under the condition of the exposure time of 1 second, and the lower right is a photograph taken under the condition of the exposure time of 2 seconds. In each photograph, the rose on the right side is a rose (with application) the petals of which have been applied with the application liquid, and the rose on the left side is a rose (without application) to which the application liquid has not been applied.

In FIG. 4, in the upper left photograph which is taken under the bright field condition, the petals exhibited a non-fluorescent white color both with and without application of the application liquid. In the upper right, lower left, and lower right photographs which are taken under the excitation light irradiation condition, the rose without application could be visually recognized with a part of the petals exhibiting a slight blue color, which was not fluoresce, and the fluorescence could not be visually recognized in the photograph in the box. In contrast, the petals of the rose with application exhibited a green fluorescent color. As Compared to Example 1-2 in which a protein liquid to which only a surfactant was added was used, Example 1-3 in which a protein liquid to which starch paste was added in addition to the surfactant was used showed more stable and intense fluorescence. From these results, it was confirmed that the fluoresce of the fluorescent protein applied to the plant can be more stably maintained by using the starch paste with the fluorescent protein.

FIG. 5 shows photographs of the Phalaenopsis orchids after the application of the application liquid. In FIG. 5, the upper left is a photograph taken under the conditions of the bright field and the exposure time of 1/6 second, the upper middle is a photograph taken under the condition of the exposure time of 4 seconds, the upper right is a photograph taken under the condition of the exposure time of 2 seconds, the lower left is a photograph taken under the conditions of the bright field and the exposure time of 1/6 second, the lower middle is a photograph taken under the condition of the exposure time of 1 second, and the lower right is a photograph taken under the condition of the exposure time of 1/2 second. In each photograph, the flowers in the right row are flowers (with application) to which the application liquid has been applied, and the flowers in the left row are flowers (without application) to which the application liquid has not been applied.

In FIG. 5, in the upper left and lower left photographs which are taken under the bright field condition, petals exhibited a non-fluorescent white color both with and without application of the application liquid. In the upper middle, upper right, lower middle, and lower right photographs which are taken under the excitation light irradiation condition, the fluorescence of the flower without application could not be visually recognized in the photograph in the box, whereas the petals of the flower with application exhibited a green fluorescent color. From these results, it was confirmed that the plant emits fluorescence by the application of the fluorescent protein.

### Example 2

It was examined that a glowing plant was obtained by causing a fluorescent protein to be absorbed inside the plant.

### (Example 2-1)

To the protein liquid (1.3 mg/ml) prepared in Example 1-1, milliQ® water was added to dilute by 5 times, thereby preparing an absorption liquid. The absorption liquid was placed in 15 ml Falcon® tube and the plant was allowed to absorb the absorption liquid from the roots for 2 days. Except for these points, the plant was photographed in the same manner as in Example 1-1.

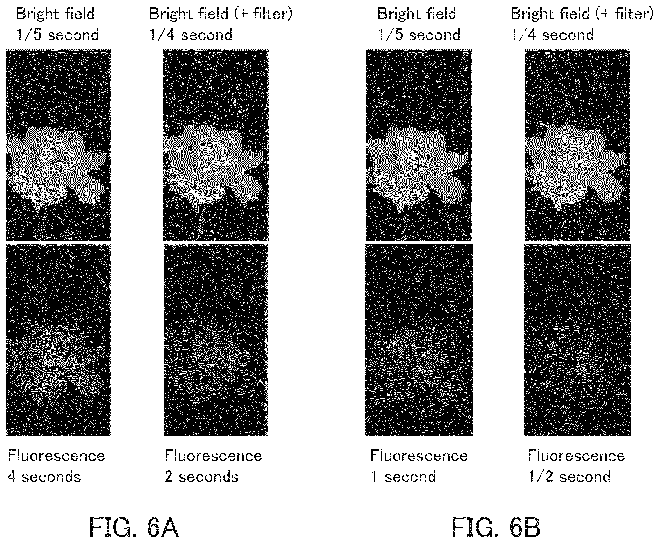

The results are shown in FIG. 6. FIG. 6 shows photographs of the roses after the absorption of the absorption liquid. In (A) of FIG. 6, the upper left is a photograph taken under the conditions of the bright field (without viewing filter) and the exposure time of 1/5 second, the upper right is a photograph taken under the conditions of the bright field (with viewing filter) and the exposure time of 1/4 second, the lower left is a photograph taken under the conditions of the excitation light irradiation and the exposure time of 4 seconds, and the lower right is a photograph taken under the conditions of the excitation light irradiation and the exposure time of 2 seconds. In (B) of FIG. 6, the upper left is a photograph taken under the conditions of the bright field (without viewing filter) and the exposure time of 1/5 second, the upper right is a photograph taken under the conditions of the bright field and the excitation light irradiation (with viewing filter) and the exposure time of 1/4 second, the lower left is a photograph taken under the conditions of the excitation light irradiation and the exposure time of 1 second, and the lower right is a photograph taken under the conditions of the excitation light irradiation and the exposure time of 1/2 second.

In each of (A) and (B) of FIG. 6, in the upper left photograph which is taken under the conditions of the bright field without the filter, the petals exhibited a non-fluorescent white color. In the upper right photograph which is taken under the conditions of the bright field with the filter, the petals exhibited a non-fluorescent yellow color due to the cutting of blue light from the light source by the viewing filter. In the lower left and lower right photographs which are taken under the excitation light irradiation condition, the petals exhibited a green fluorescent color. From these results, it was confirmed that the plant emits fluorescence by the absorption of the fluorescent protein.

While the present invention has been described above with reference to illustrative example embodiments and examples, the present invention is by no means limited thereto. Various changes and variations that may become apparent to those skilled in the art may be made in the configuration and specifics of the present invention without departing from the scope of the present invention.

This application claims priority from Japanese Patent Application No. 2016-173930 filed on September 6, 2016. The entire subject matter of the Japanese Patent Application is incorporated herein by reference.

### Industrial applicability

According to the glowing plant of the present invention, for example, a plant that emits fluorescence can be obtained without using a gene recombination technique for the plant. In addition, since the fluorescent protein is applied to plants, for example, the affinity to plants is higher and the safety to plants and environments is also higher as compared to the case of applying an organic synthetic fluorescent compound as described above. Therefore, the present invention is extremely useful in the field of horticulture and the like.

### [Sequence listing]

TF16085WO_ST25.txt

## Claims

1. A glowing plant comprising:
a fluorescent protein applied to a surface of a plant; or
a fluorescent protein absorbed inside the plant.

2. The glowing plant according to claim 1, wherein
the plant is a flower.

3. The glowing plant according to claim 1 or 2, comprising:
a fluorescent protein absorbed from a root or a stem.

4. The glowing plant according to any one of claims 1 to 3, wherein
the fluorescent protein applied to the surface of the plant is a fluorescent protein-containing substance or the fluorescent protein absorbed by the plant is a fluorescent protein-containing substance, and
the fluorescent protein-containing substance is a homogenate of a host expressing the fluorescent protein.

5. The glowing plant according to claim 4, wherein
the fluorescent protein-containing substance is an individual-removed substance obtained by removing an individual of the host from the homogenate.

6. The glowing plant according to any one of claims 1 to 3, wherein
the fluorescent protein applied to the surface of the plant is a fluorescent protein-containing substance or the fluorescent protein absorbed by the plant is a fluorescent protein-containing substance, and
the fluorescent protein-containing substance is a culture of a host expressing the fluorescent protein.

7. The glowing plant according to claim 6, wherein
the fluorescent protein-containing substance is a supernatant of the culture.

8. The glowing plant according to any one of claims 4 to 7, wherein
the host is a microorganism or a virus.

9. A method of producing a glowing plant, comprising the step of:
applying a fluorescent protein to a surface of a plant; or
causing a fluorescent protein to be absorbed inside the plant.

10. The method according to claim 9, wherein
the plant is a flower.

11. The method according to claim 9 or 10, wherein
in the absorption step, a root or a stem of the plant is brought into contact with water that contains the fluorescent protein to cause the fluorescent protein to be absorbed from the root or stem.

12. The method according to any one of claims 9 to 11, wherein
in the application step, a fluorescent protein-containing substance that contains the fluorescent protein is applied to the surface of the plant, or
in the absorption step, the fluorescent protein-containing substance that contains the fluorescent protein is caused to be absorbed by the plant, and
the fluorescent protein-containing substance is a homogenate of a host expressing the fluorescent protein.

13. The method according to claim 12, wherein
the fluorescent protein-containing substance is an individual-removed substance obtained by removing an individual of the host from the homogenate.

14. The method according to claim 13, further comprising the step of:
filtrating the homogenate with a filter through which the host does not pass to obtain the individual-removed substance.

15. The method according to any one of claims 9 to 11, wherein
in the application step, a fluorescent protein-containing substance that contains the fluorescent protein is applied to the surface of the plant, or
in the absorption step, the fluorescent protein-containing substance that contains the fluorescent protein is caused to be absorbed by the plant, and
the fluorescent protein-containing substance is a culture of a host expressing the fluorescent protein.

16. The method according to claim 15, wherein
the fluorescent protein-containing substance is a supernatant of the culture.

17. The method according to any one of claims 9 to 16, further comprising the step of:
purifying the fluorescent protein from the fluorescent protein-containing substance, wherein
in the application step, the purified fluorescent protein is applied.

18. The method according to claim 17, wherein
in the purification step, the fluorescent protein is purified using a column.

19. The method according to any one of claims 12 to 18, wherein
the host is a microorganism or a virus.
